# EUROPÄISCHE PATENTSCHRIFT

(11) **EP 1 713 933 B1**
(45) Veröffentlichungstag und Bekanntmachung des Hinweises auf die Patenterteilung: **30.07.2014**
(21) Anmeldenummer: 05701315.3
(22) Anmeldetag: 02.02.2005
(51) Int. Cl.: C12Q 1/68, G01N 33/58

(54) **VESIKEL ZUM ABTRENNEN VON SUBSTANZEN AUS FLÜSSIGEN MEDIEN**
VESICLE USED TO SEPARATE SUBSTANCES FROM LIQUID MEDIA
VESICULE PERMETTANT LA SEPARATION DE SUBSTANCES CONTENUES DANS DES MILIEUX LIQUIDES

(30) Priorität: 02.02.2004 EP 04002214
(43) Veröffentlichungstag der Anmeldung: 25.10.2006
(73) Patentinhaber: RWTH Aachen, 52062 Aachen (DE); Universität Basel, 4003 Basel (CH)
(72) Erfinder: SCHWANEBERG, Ulrich, 27721 Ritterhude (DE); WINTERHALTER, Mathias, 28757 Bremen (DE); MEIER, Wolfgang, CH-4106 Therwil (CH)
(74) Vertreter: Jeschke, Alexander
(86) Internationale Anmeldenummer: PCT/EP2005/001039
(87) Internationale Veröffentlichungsnummer: WO 2005/073402

(56) Entgegenhaltungen:
- WO-A-00/32812
- WO-A-01/32146
- WO-A-03/106589
- NARDIN CORINNE ET AL: "Hybrid materials from amphiphilic block copolymers and membrane proteins." JOURNAL OF BIOTECHNOLOGY. MAR 2002, Bd. 90, Nr. 1, März 2002 (2002-03), Seiten 17-26, XP009040280 ISSN: 0168-1656 in der Anmeldung erwähnt

## Beschreibung

Die Erfindung betrifft Verfahren zum Abtrennen von Substanzen aus flüssigen Medien und dazu geeignete Trennmittel.

Zum Abtrennen von Substanzen aus flüssigen Medien sind eine Reihe von Verfahren und Trennmitteln bekannt. Besondere Bedeutung im Bereich der Biochemie haben adsorptive Verfahren erlangt wie die AdsorptionsChromatographie, lonenaustausch-Chromatographie und dergleichen. Bei adsorptiven Verfahren bindet die abzutrennende Substanz an ein Trägermaterial, zumeist eine festen Phase, und wird zusammen mit dem Trägermaterial vom flüssigen Medium abgetrennt. Üblicherweise verwendet werden Trägermaterialien mit einer hohen spezifischen Oberfläche, insbesondere Glasperlen und Polymerperlen. Zum Vergrößern der Oberfläche können die Trägermaterialien zudem porös sein, so dass auch im Inneren des Trägermaterials Oberflächen zum Binden der abzutrennende Substanz zur Verfügung gestellt werden. Um eine möglichst gute Diffusion der abzutrennenden Substanz zum Inneren eines porösen Trägers zu gewährleisten und damit eine gute Adsorptionsleistung zu ermöglichen, wird der Porenquerschnitt groß gewählt, so dass der Stofftransport - insbesondere durch Diffusion - ins Trägerinnere nicht oder nur unwesentlich behindert wird. Üblicherweise betragen die durchschnittlichen Porendurchmesser daher ein Vielfaches der Größe der abzutrennenden Substanz.

Herkömmliche Trennverfahren ermöglichen es jedoch nur mit großem Aufwand, aus einer Mischung mehrerer chemisch ähnlicher Substanzen eine bestimmte Substanz oder eine bestimmte Gruppe von Substanzen abzutrennen. Dazu werden häufig mit Antikörpern oder deren Bindungsstellen versehene "Perlen" verwendet. Diese sind jedoch aufwendig und verhältnismäßig teuer herzustellen, da zunächst die entsprechenden Antikörper erzeugt werden müssen. Im übrigen sind sie nur sehr beschränkt geeignet, Nukleinsäuren voneinander zu unterscheiden und selektiv aus einem flüssigen Medium abzutrennen.

Es war daher eine Aufgabe der vorliegenden Erfindung, Trennmittel zum Abtrennen einer Substanz aus einem flüssigen Medium anzugeben. Die Trennmittel sollten möglichst einfach herstellbar sein, das selektive Abtrennen einer möglichst großen Vielfalt verschiedener Substanzen ermöglichen, sie sollten stabil sein in einer Vielzahl üblicher flüssiger Medien wie Zellkulturmedien und Zellextrakten, und sie sollten einen möglichst hohen Anteil der im Medium vorliegenden abzutrennenden Substanz abtrennen können. Ferner sollten entsprechende Trennverfahren bzw. Verwendungen der Trennmittel angegeben werden.

Die Aufgabe wird gemäß einem Aspekt gelöst durch Vesikel
- mit einer Membran enthaltend amphiphile Moleküle,
- einer in der Membran enthaltenen porenbildenden Poreneinheit, um den Zugang zum Vesikel-Inneren zu ermöglichen,
wobei das Vesikel im Vesikel-Inneren eine Bindungssubstanz enthält zum Binden der zu bindenden Substanz, und wobei die Bindungssubstanz nicht durch die von der Poreneinheit gebildete Pore diffundieren kann.

Die Veröffentlichung WO 03/106589 A1 offenbart nanostrukturierte Materialien zum Abtrennen von Stoffen aus einer Lösung. Die nanostrukturierten Materialien werden als Partikel dispergiert und sind durch Nanostrukturierung ihrer Oberfläche porös. Die Veröffentlichung offenbart jedoch keine in einer Membran enthaltenen porenbildende Poreneinheit. Sie betrifft zudem nicht das technische Gebiet der Vesikel, sondern das der nanostrukturierten Materialien.

Durch das Einbringen bzw. Vorsehen einer im Vesikel-Inneren eingeschlossenen Bindungssubstanz wird es erstmals möglich, dauerhaft größere Mengen allein durch chemische Bindung bzw. chemische Wechselwirkungen im Inneren des Vesikels zu binden. Dies ermöglicht es beispielsweise. Nukleinsäuren oder andere geladene Makromoleküle auch bei geöffneten Poren lange im Inneren eines solchen Vesikels zu halten. Damit eignet sich das erfindungsgemäße Vesikel insbesondere als Trennmittel zum Abtrennen einer Substanz aus einem flüssigen Medium, indem die Bindungssubstanz so ausgewählt wird, das die abzutrennende Substanz von der Bindungssubstanz gebunden wird, so dass die gebundene Substanz mit dem Vesikel selbst vom flüssigen Medium abgetrennt werden kann, beispielsweise durch Filtration oder Sedimentation/Zentrifugation. Im Gegensatz zu herkömmlichen porösen Trennmitteln ist den erfindungsgemäßen Vesikeln zudem von vornherein eine Spezifität für solche Substanzen zu eigen, die die von der Poreneinheit gebildete Pore passieren können und eine Affinität an die Bindungssubstanz haben. Die erfindungsgemäßen Vesikel können daher beispielsweise ähnliche Substanzen wie PCR-Primer oder doppelsträngige PCR-Produkte von größeren Nukleinsäuren wie beispielsweise Plasmid-DNA trennen.

Vorzugsweise enthält die Membran als amphiphiles Molekül ein amphiphiles Copolymer mit einem hydrophilen und einem hydrophoben Abschnitt. Insbesondere ist es bevorzugt, wenn die Membran des erfindungsgemäßen Vesikels aus amphiphilen Block-Copolymer-Molekülen gebildet wird, wobei es insbesondere bevorzugt ist, wenn die Membran aus einer einzigen Art von amphihilen Block-Copolymer-Molekülen gebildet wird.

Vesikel aus amphiphilen Block-Copolymeren sind bereits zuvor als Bioreaktoren beschrieben worden (Nardin et al., Eur. Phys. J. E 4, (2001), 403-410, "Amphiphilic block copolymer nanocontainers as bioreactors"; sowie WO 01/32146). Dabei handelt es sich um Vesikel oder Micellen aus aggregierenden Block-Copolymeren mit zumindest einem hydrophilen und einem hydrophoben Abschnitt. Die entstehenden Vesikel/Micellen sind sehr stabil gegenüber Verdünnung, können eine monodisperse Größenverteilung besitzen und weisen eine sehr niedrige Dynamik auf, so dass ihre Halbwertszeit bis zu einigen Stunden betragen kann. Die beschriebenen Bioreaktoren sind jedoch ungeeignet zum Konzentrieren und Abtrennen von Substanzen. Insbesondere können Substanzen mit einem hohen Molekulargewicht von z.B. mehr als 1000 Da nicht in den Bioreaktor hinein diffundieren. Ferner besitzen die Bioreaktoren keine Mittel, um Substanzen in ihrem Inneren in höherer Konzentration aufzunehmen als in der Lösung. Zudem werden die in den Bioreaktor aufgenommenen Substanzen zu Produkten umgesetzt, die entsprechend einem Konzentrationsgradienten den Bioreaktor wieder verlassen.

Ferner sind Vesikel bekannt, bei denen ein Polymer-Gerüst im Inneren der Membran eines Liposoms durch Polymerisation aufgebaut wird (Hotz und Meier, Langmuir 1998, 14, 1031 - 1036, "Vesicle-Templated Polymer Hollow Spheres"). Diese können auch nach Entzug der zur Polymerisation verwendeten oberflächenaktiven Substanzen im trockenen Zustand stabile Hohlkugeln bilden.

Zur Herstellung und Verarbeitung der Vesikel und insbesondere der in der Vesikel-Membran enthaltenen amphiphilen Copolymere wird der Fachmann insbesondere die oben bezeichneten Veröffentlichungen von C. Nardin et al. (Eur. Phys. J. E 4, (2001), 403-410) sowie die internationale Patentanmeldung WO 01/32146 heranziehen. Die darin beschriebenen Ausführungsformen entsprechender Vesikel-Membranen und der in ihnen enthaltenen amphiphilen Copolymere sind auch erfindungsgemäß besonders bevorzugt.

Insbesondere ist es bevorzugt, wenn das amphiphile Copolymer ein segmentiertes Copolymer ist mit zumindest einem hydrophilen Abschnitt A und zumindest einem hydrophoben Abschnitt B, wobei sich die segmentierten Copolymere selbst unter Bildung von Vesikeln zusammenlagern können. Das amphiphile Copolymer kann auch mehr als einen hydrophilen und mehr als einen hydrophoben Abschnitt enthalten, insbesondere kann das Copolymer eine ABA-Struktur mit zwei hydrophilen und einem dazwischen angeordneten hydrophoben Abschnitt besitzen. Zur Bildung erfindungsgemäßer Vesikel bevorzugt sind Block-Copolymere, wobei es sich insbesondere um lineare Block-Copolymere handeln kann. Anstelle oder ergänzend zu linearen Block-Copolymeren können jedoch auch gepfropfte Copolymere oder Kammstrukturen verwendet werden, die sowohl (zumindest) einen hydrophilen als auch (zumindest) einen hydrophoben Abschnitt besitzen.

Wird ein ABA-Copolymer zum Bilden eines erfindungsgemäßen Vesikels verwendet, dann bildet sich ein Vesikel mit einer hydrophilen Innen- und Außenschicht sowie einer hydrophoben Zwischenschicht. Wird ein BAB-Copolymer verwendet, so bildet sich ein Vesikel mit hydrophober Innen- und Außenschicht sowie einer hydrophilen Zwischenschicht. Weitere bevorzugte Copolymere und deren Verwendungsmöglichkeiten zum Bilden von Vesikeln können insbesondere der WO 01/32146 entnommen werden.

In einer weiteren bevorzugten Ausführungsform der erfindungsgemäßen Vesikel enthält die Membran mehrere der amphiphilen Copolymere, wobei die amphiphilen Copolymere miteinander kovalent verbunden sind. Die Copolymere können insbesondere durch Polymerisation miteinander verbunden werden. Beispiele solcher erfindungsgemäß bevorzugten Copolymere und daraus gebildete Vesikel-Membranen sind ebenfalls der WO 01/32146 bzw. der genannten Veröffentlichung von C. Nardin et al. zu entnehmen.

Dementsprechend besonders bevorzugt enthält die Membran ein Poly-(2-methyloxazolin)-Block-Poly (dimethylsiloxan)-Block-Poly (2-methyl-oxazolin)-Triblock-Copolymer (PMOXA-PDMS-PMOXA), vorzugsweise mit einer oder mehreren polymerisierbaren Gruppen an beiden Kettenenden, wie auch in Beispiel 1 der WO 01/32146 beschrieben. Dieses Copolymer ist besonders geeignet zur Bildung stabiler Vesikel und erleichtert den Einbau einer porenbildenden Poreneinheit in die Membran.

Es ist nicht notwendig, dass die Membran des erfindungsgemäßen Vesikels lediglich aus amphiphilen Copolymeren einer einzigen Art aufgebaut ist. Insbesondere kann die Membran außer PMOXA-PDMS-PMOXA-Triblock-Copolymeren noch weitere Arten amphiphiler Copolymere enthalten.

Die Bindungssubstanz bindet an die zu bindende Substanz über eine chemische Bindung, also unter Ausbildung einer Hauptvalenzbildung - beispielsweise einer kovalenten Bindung oder einer ionischen Bindung -, einer Nebenvalenzbindung - beispielsweise eine Van der Waals-Wechselwirkung und/oder eine Wasserstoffbrückenbindung -, und/oder durch eine hydrophile oder hydrophobe Wechselwirkung. Besonders bevorzugt ist es, wenn die Bindungssubstanz eingerichtet ist zum Ausbilden einer oder mehrerer Wasserstoffbrücken, Ionenbindungen und/oder zur hydrophoben Wechselwirkung mit der zu bindenden Substanz. Diese Bindungsarten ermöglichen ein spezifisches Binden an zahlreiche in der biochemischen Praxis relevante Substanzen; sie ermöglichen es außerdem, die gebundene Substanz mit einfachen Mitteln wieder von der Bindungssubstanz zu lösen. In besonders bevorzugten Ausführungsformen sind die erfindungsgemäßen Vesikel eingerichtet, um die abzutrennende Substanz in ihrem Inneren im Vergleich zu einer umgebenden Lösung anzureichern.

Die im Vesikel-Inneren enthaltene Bindungssubstanz ist vorzugsweise ein Polyelektrolyt. Dabei ist es bevorzugt, als Bindungssubstanz eine Polysäure zu verwenden, wenn das Vesikel-Innere eine oder mehrere positiv geladene Substanzen binden soll. Beispiele geeigneter Polysäuren sind Polyphosphorsäure, Polyvinylschwefelsäure, Polyvinylsulfonsäure, Polyvinylphosphonsäure und Polyacrylsäure. Soll das Vesikel-Innere jedoch eine oder mehrere negativ geladene Substanzen binden, so ist dieses vorzugsweise einer Polybase ausgerüstet, beispielsweise Polyethylenimin, Polyvinylamin, Polyvinylpyridin und/oder Polylysin.

Ergänzend oder alternativ dazu kann die Bindungssubstanz auch eine Oligosäure bzw. eine Oligobase aus 10 - 100 Säure- bzw. Basegrundeinheiten sein. Der Fachmann kann entsprechende Oligo- und Polysäuren bzw. -basen insbesondere danach auswählen, ob sie durch eine Pore der porenbildenden Einheit hindurch diffundieren kann, wobei es zweckmäßig ist, solche Bindungssubstanzen auszuwählen, die zu einer solchen Diffusion nur in sehr geringem Maße in der Lage sind.

Ergänzend oder alternativ zur Verwendung von Polysäuren bzw. Oligosäuren bzw. -basen kann im Vesikel-Innere eine Affinität für die zu bindende Substanz auch durch eine nach der Vesikelbildung erfolgende Reaktion unter Knüpfung und/oder Auflösung einer kovalenten Bindung von in der Vesikel-Membran enthaltenen Einheiten gebildet werden.

Zudem ist es besonders bevorzugt, wenn das Vesikel-Innere im wesentlichen hohl ist. Dies ermöglicht es, im wesentlichen das gesamte von der Vesikel-Membran umschlossene Volumen für das Binden der zu bindenden Substanz zu nutzen, Darüber hinaus wird durch die Vesikel-Membran eine gebundene Substanz geschützt; beispielsweise können Proteasen aus einem umgebenden Medium nicht mehr in das Vesikel-Innere gelangen.

Ebenfalls besonders bevorzugt ist es, wenn die Vesikel-Membran auf ihrer Außenseite frei ist von der Bindungssubstanz. Die zu bindende Substanz wird dann hauptsächlich über die im Vesikel-Inneren vorliegende Bindungssubstanz gebunden; dies ermöglicht es, die durch die Poreneinheit erzielbare besonders gute Spezifität zum Unterscheiden ähnlicher Substanzen (beispielsweise kurzer PCR-Produkte von großen Plasmiden) zu nutzen.

Die Poreneinheit ist vorzugsweise eingerichtet, die Permeation von Substanzen (vorzugsweise insbesondere der zu bindenden Substanz) in das Vesikel-Innere zu regulieren.

Besonders bevorzugt sind solche Vesikel, deren Poreneinheit ein Protein oder einen Proteinteil enthält, das/der ausgewählt ist aus der Gruppe bestehend aus
a) einem porenbildenden Transmembranprotein,
b) einem porenbildenden Transmembranprotein mit einer alpha-helikalen Transmembran-Struktur, insbesondere Alamethicin, Melittin, Magainin, Dermaseptin,
c) einem porenbildenden Transmembranprotein mit einer *β*-Fass-Transmembran-Struktur, insbesondere *Rhodobacter capsulatus*-Porin, *Rhodopseudomonas blastica*-Porin, Omp, ScrY, FepA, PhoE sowie insbesondere für Substanzen mit einem Molekulargewicht ≤ 1000 Da OmpF, LamB, OmpK36 und für Substanzen mit einem Molekulargewicht > 1000 Da FhuA, TolC, Maltoporin und alpha-Hämolysin,
d) einer Transmembran-Struktur eines porenbildenden Transmembranproteins, und
e) einem Protein, dass eine zu einer porenbildenden Transmembranstruktur eines der Proteine gemäß a), b), c) und/oder d) strukturhomologe Struktur besitzt.

Nähere Informationen zu den soeben aufgezählten Proteinen entnimmt der Fachmann den entsprechenden Veröffentlichungen von Alberts et al., Molecular Biology of the Cell, Garland Publishing, Inc., 3. Aufl., 1994, S. 485-498 nebst weiteren Nachweisen; Biggin PC und Sansom MS, Biophys. Chem. 1999, 76(3): 161-183; Pawlak et al., Protein Sci. 1994, 3(10): 1788-1805, Template assembled melittin: structural and functional characterization of a designed, synthetic channel-forming protein; Sakai N und Matile S, Chem Commun (Camb.) 2003, (20): 2514-2523, Synthetic multifunctional pores: lessons from rigid-rod beta barrels; Weiss et al., FEBS Lett. 280 (1991), The structure of porin from *Rhodobacter capsulatus* at 1.8 Å resolution; Kreusch et al., Protein Sci. 3 (1994): 58-63, Structure of the membrane channel porin from *Rhodopseudomonas blastica* at 2.0 Å resolution; Cowan et al., Nature 358 (1992): 727-733, Crystal structures explain functional properties of two *E. coli* porins; Dutzler et al., Struct. Fold. Des. 7 (1999): 425-434, Crystal structure and functional characterization of OmpK36, the osmoporin of *Klebsiella pneumoniae*; Zeth et al., Structure Fold Des. 8 (2000): 981-992, Crystal structure of Omp32, the anion-selective porin from *Comamonas acidovorans,* in complex with a periplasmic peptide at 2.1 Å resolution; Schirmer et al., Science 267 (1995): 512-514, Structural basis for sugar translocation through maltoporin channels at 3.1 Å resolution; Danelon et al., J Biol Chem. 2003, 278(37): 35542-35551, Probing the orientation of reconstituted maltoporin channels at the single-protein level; Forst et al., Nat. Struct. Biol. 5 (1998): 37-46, Structure of the sucrose-specific porin ScrY from *Salmonella typhimurium* and its complex with sucrose; Buchanan et al., Nat. Struct. Biol. 6 (1999): 56-63, Crystal structure of the outer membrane active transporter FepA from *Escherichia coli;* Fergusson et al., Science 282 (1998): 2215-2220, Siderophore-mediated iron transport: crystal structure of FhuA with bound lipopolysaccharide; Locher et al., Cell 95 (1998): 771-778, Transmembrane signalling across the ligand-gated FhuA receptor: crystal structures of free and ferrichrome-bound states reveal allosteric changes; Koronakis et al. und Song et al. wie oben angegeben.

Dabei wird eine Struktur als strukturhomolog zu einer Ausgangsstruktur angesehen, wenn sie ebenso wie die Ausgangsstruktur das Bilden einer Pore in einer Vesikel-Membran ermöglicht.

Die Permeation von Substanzen in das Vesikel-Innere wird in bevorzugten Ausführungsformen der Erfindung durch die Poreneinheit insbesondere dadurch reguliert, dass die Poreneinheit Substanzen mit einer vorgewählten Mindest- und/oder Höchstgröße und/oder einer vorgewählten Mindest-und/oder Höchstladungsdichte den Durchtritt durch die Vesikel-Membran ermöglicht. Durch die Verwendung einer Poreneinheit wird es auf vorteilhaft einfache Weise ermöglicht, eine gleichbleibende, vorwählbare Porengröße einzustellen sowie die zum sonstigen Regulieren der Permeation notwendigen Poreneigenschaften (insbesondere Ladungsverteilungen) gleichbleibend und vorwählbar bereitzustellen und mittels molekularbiologischer Methoden (z.B. Mutagenese) gezielt zu verändern, um beispielsweise Selektivitäten und Transporteigenschaften zu ändern.

In bevorzugten Vesikeln enthält die Poreneinheit ein Protein oder einen Proteinteil, das/der eine *β*-Fass-Struktur enthält. Eine *β*-Fass-Struktur tritt sehr häufig in integralen Membranproteinen auf. Sie ist insbesondere ein häufiges Merkmal von Proteinen, die am Stofftransport durch die äußere bakterielle Membran beteiligt sind. Besonders gut charakterisiert und erfindungsgemäß als Poreneinheit bevorzugt sind Porine bzw. deren jeweilige *β*-Fass-Domäne oder deren *β*-Fass-Struktur.

Die Poreneinheit muss nicht ausschließlich aus einem Protein bestehen, sondern kann auch aus zwei oder mehr Proteineinheiten gebildet werden, die zusammenwirken, um eine Pore zu bilden. Vorzugsweise bilden die Teilproteine in der Poreneinheit eine die Membran durchdringende Wand mit insgesamt gesehen einer *β*-Fass-Struktur. Beispiele solcher ebenfalls bevorzugter Poreneinheiten, deren *β*-Fass-Struktur aus dem Zusammenwirken mehrere Proteineinheiten hervorgeht, sind das trimere TolC-Protein aus *E. coli* und das heptamere Haemolysin-Toxin der Staphylokokken (s. Koronakis et al.; Nature 405 (2000): 914-919; Crystal structure of the bacterial membrane protein TolC central to multidrug efflux and protein export, bzw. Song et al., Science 274 (1996): 1859-1866, Structure of staphylococcal *α*-hemolysin, a heptameric transmembrane pore). Soweit im Rahmen des vorliegenden Textes von einer *β*-Fass-Struktur gesprochen wird, wird darunter auch immer ein solcher Abschnitt eines Proteins verstanden, der, wenn er mit geeigneten weiteren Abschnitten gegebenenfalls weiterer Proteine zusammentritt, in einer Membran eines erfindungsgemäßen Vesikels eine Pore mit *β*-Fass-Struktur bildet.

Eine besonders bevorzugte Poreneinheit ist das FhuA-Kanalprotein bzw. ein Protein mit dessen *β*-Fass-Domäne oder mit einer zu dieser strukturhomologen Struktur. Dieses Protein ist sehr temperaturstabil (bis ca. 60° C), es erlaubt den Transport von Phagen-DNA in das Vesikel-Innere und ist in üblichen Wirtszellen exprimierbar, beispielsweise in *E. coli.* Das Protein besitzt einen im Wesentlichen elliptischen Porendurchmesser von 4,6 nm zu 3,9 nm. Die *β*-Fass-Domäne wird dabei von 22 antiparallelen Faltblättern gebildet.

Ein Beispiel eines Proteins mit der *β*-Fass-Struktur von FhuA ist ein FhuA-Derivat, das hervorgegangen ist durch Entfernen der Aminosäuren 5 - 160 von FhuA (s. Braun et al., Eur. J. Biochem. 269 (2002): 4948-4959, Diffusion through channel derivatives of the *Escherichia coli* FhuA transport protein). Dieses FhuA-Derivat ist als Poreneinheit besonders bevorzugt. Es bildet eine Pore mit einem Innendurchmesser, der an der engsten Stelle etwa 1 nm breit ist. Durch geeigneten Aminosäureaustausch kann diese Porenweite verengt, erweitert oder die Pore auf ihrer Poren-Innenseite mit Ladungen versehen werden.

Ferner ist es bevorzugt, wenn die Poreneinheit einen inneren Porendurchmesser von mehr als 0,1 nm, vorzugsweise mindestens 1 nm und besonders bevorzugt mindestens 3,5 nm Breite besitzt. Poreneinheiten mit entsprechenden Porendurchmessern lassen sich besonders leicht anhand von *β*-Fass-Strukturen bekannter Proteine erzeugen, wie soeben am besonders bevorzugten Beispiel des FhuA-Proteins dargestellt. Zudem ermöglichen entsprechende Poreneinheiten nicht nur kleinen Atomen und Molekülen den Zutritt ins Innere der erfindungsgemäßen Vesikel, sondern auch größeren . Molekülen, insbesondere Nukleinsäuren wie DNA und RNA. Entsprechende Poreneinheiten werden daher erfindungsgemäß bevorzugt verwendet zum Abtrennen einer Nukleinsäure, insbesondere einer DNA und/oder einer RNA.

Ferner ist es erfindungsgemäß bevorzugt, wenn die Poreneinheit einen inneren Porendurchmesser von nicht mehr als 10 nm Breite, vorzugsweise nicht mehr als 8 nm und besonders bevorzugt nicht mehr als 5 nm Breite besitzt. Die Poreneinheit erlaubt so auf vorteilhaft einfache Weise, die erfindungsgemäßen Vesikel nach Art eines Siebes zu verwenden und die Substanzen, die ins Vesikel-Innere gelangen können, anhand ihrer Breite auszuwählen.

Sollen Nukleinsäuren, insbesondere einzel- und/oder doppelsträngige DNA bzw. RNA in dem erfindungsgemäßen Vesikel aufgenommen werden, so beträgt der Porendurchmesser vorzugsweise zumindest 1 nm und nicht mehr als 5 nm.

Ferner sind erfindungsgemäß besonders solche Vesikel bevorzugt, die im Vesikel-Inneren ein positiv geladenes Oligomer und/oder Polymer besitzen. Beispiele entsprechender Oligomere und Polymere sind bereits weiter oben beschrieben worden. Erfindungsgemäß besonders bevorzugt sind dabei solche Vesikel, die im Vesikel-Inneren Polylysin enthalten. Mit entsprechenden Oligomeren und Polymeren, insbesondere mit Polylysin, können sehr einfach erfindungsgemäße Vesikel gebildet werden, die sich besonders eignen zur Aufnahme und zum Binden von Nukleinsäuren, insbesondere einzel- und doppelsträngiger DNA bzw. RNA im Vesikel-Inneren. Dabei wird zweckmäßigerweise im Vesikel-Inneren eine ausreichend hohe Konzentration des positiv geladenen Oligo- und/oder Polymers bereitgestellt (insbesondere an Polylysin), um ein sicheres Festhalten der im Vesikel aufzunehmenden Nukleinsäure (insbesondere einzel- und/oder doppelsträngiger DNA oder RNA) zu gewährleisten.

Demgemäß ist es ferner bevorzugt, erfindungsgemäße Vesikel zum Binden einer geladenen Substanz zu verwenden. Aufgrund ihrer besonderen; oben beschriebenen Struktur sind erfindungsgemäße Vesikel besonders geeignet, eine oder mehrere elektrisch geladene Substanzen in ihrem Inneren aufzunehmen. Durch. Verwendung geeigneter, beispielsweise eine Potentialdifferenz hervorrufende Substanzen können die erfindungsgemäßen Vesikel besonders gut zum Festhalten und Binden einer oder mehrerer elektrisch geladener Substanzen in ihrem Inneren verwendet werden. Durch eine geeignete Auswahl eines Porenbildners können die erfindungsgemäßen Vesikel zudem dazu verwendet werden, aus einer Mischung unterschiedlicher elektrisch geladener Substanzen nur solche mit einer vorgewählten Höchstgröße aufzunehmen und gegebenenfalls zu binden. Besonders geeignete Porenbildner wurden oben bereits beschrieben; besonders bevorzugt ist ein FhuA-Protein bzw. ein Protein mit der *β*-Fass-Domäne von FhuA als Porenbildner in einem erfindungsgemäßen Vesikel zum Aufnehmen und gegebenenfalls zum Binden einer elektrisch geladenen Substanz.

Es ist ebenfalls bevorzugt, als Poreneinheit ein Protein bzw. Teilprotein einer der oben beschriebenen Arten auszuwählen, die im Poreninneren eine positive oder eine negative Nettoladung besitzt. Eine solche Pore ermöglicht es; den Durchtritt durch die Pore für solche Substanzen zu erleichtern, die eine entgegengesetzte Ladung besitzen, bzw. für solche Substanzen zu erschweren, die die gleiche Ladung besitzen. Insbesondere kann auf diese Weise eine geladene Bindungssubstanz auch dann im Vesikel-Inneren festgehalten werden, wenn diese allein aufgrund ihrer Größe ansonsten durch die vom Porenbildner gebildete Pore hindurchdiffundieren könnte.

Zudem ist es bevorzugt, wenn die Poreneinheit eine enantioselektive Pore bildet. Ein Beispiel für eine enantioselektiven Poreneinheit ist Maltoporin (Danelon et al., s.o.). Darüber hinaus können durch Einbauen geeigneter Aminosäuren auch zuvor nicht enantioselektive Transmembranproteine bzw. deren Transmembran-Strukturen enantioselektiv gemacht werden. Ferner ist zu erwarten, dass weitere enantioselektive Transmembranproteine gefunden werden. Die Verwendung eines enantioselektiven Transmembranproteins bzw. einer enantiomeren Transmembran-Suktur in einer Poreneinheit (bzw. als Poreneinheit) eines Vesikels mit einer Membran enthaltend amphiphile Copolymere ist ebenfalls bevorzugt.

Ferner ist es besonders bevorzugt, wenn die im Vesikel aufzunehmende und/oder zu bindende Substanz eine Nukleinsäure ist. Die erfindungsgemäßen Vesikel ermöglichen es bei einer geeigneten Wahl der Poreneinheit, Nukleinsäuren auf einfache Weise und mit hoher Effizienz aus flüssigen Mischungen abzutrennen, insbesondere von Proteinen.

Die Ladungen im Inneren der erfindungsgemäßen Vesikel können in bevorzugten Ausführungsformen insbesondere über den pH-Wert, über die Salzkonzentration oder die Anzahl der ionischen Gruppen in den Vesikeln moduliert und an vielfältige Problemstellungen angepasst werden.

Aus den erfindungsgemäßen Vesikeln können die darin aufgenommenen und/oder darin gebundenen Substanzen durch a) mechanisches Zerstören der Vesikel, beispielsweise durch Anlegen einer Scherkraft, b) durch Auflösen der Vesikel, beispielsweise in Ethanol, und/oder c) durch Zugabe eines Salzes, beispielsweise NaCl, wieder herausgelöst werden.

Dementsprechend bevorzugt ist es, die erfindungsgemäßen Vesikel zum Aufnehmen und/oder zum Binden einer Nukleinsäure zu verwenden. Eine solche Verwendung der erfindungsgemäßen Vesikel erlaubt insbesondere ein schnelles Abtrennen einer Nukleinsäure aus einer Mischung enthaltend weitere geladene Substanzen, beispielsweise Proteine. Die Verwendung der erfindungsgemäßen Vesikel zu diesem Zweck ermöglicht es ebenfalls, Nukleinsäuren mit hoher Ausbeute aus entsprechenden Mischungen zu gewinnen. Zudem wird für die Nukleinsäure-Gewinnung in besonders bevorzugten Ausführungsformen keine Agarose benötigt und schließlich fallen bei der erfindungsgemäßen Verwendung nur geringe Mengen an Abfällen an, wobei weitgehend auf kanzerogene Reagenzien verzichtet werden kann.

Es ist deshalb auch ein Verfahren zum Abtrennen einer Nukleinsäure aus einer nukleinsäurehaltigen Lösung bevorzugt, umfassend das Inkontaktbringen der abzutrennenden Nukleinsäure mit einem erfindungsgemäßen Vesikel wie oben beschrieben. Dabei ist es zweckmäßig, erfindungsgemäße Vesikel zu verwenden, die im Vesikel-Inneren ein positiv geladenes Oligomer und/oder Polymer besitzen. Besonders bevorzugt ist es, polylysinhaltige Vesikel zu verwenden. Die Porengröße der verwendeten erfindungsgemäßen Vesikel beträgt in bevorzugten Ausführungsformen des Verfahrens 1 - 5 nm; wobei als Poreneinheit besonders bevorzugt ein FhuA-Derivat ist, dem im Vergleich zum nativen FhuA-Protein die Aminosäuren 5-160 fehlen.

Das erfindungsgemäße Verfahren, insbesondere unter Verwendung der soeben beschriebenen bevorzugten erfindungsgemäßen Vesikel einschließlich der besonders bevorzugten Poreneinheiten, ermöglicht es auch, Nukleinsäuren unterschiedlicher Größe voneinander zu trennen. Beispielsweise können große Nukleinsäuren wie Plasmid-DNA bei entsprechender Auswahl der Poreneinheit (insbesondere eines Poreneinheit mit einer *β*-Fass-Struktur, die strukturhomolog ist zu der von FhuA) aufgrund ihrer Breite nicht ins Innere der erfindungsgemäßen Vesikel gelangen, während dies für kürzere, nicht zirkuläre und insbesondere einzelsträngige Nukleinsäuren möglich ist. Aufgrund dieser Siebwirkung der Poreneinheit können mit dem erfindungsgemäßen Verfahren zudem Nukleinsäuren schnell und mit hoher Ausbeute von Proteinen abgetrennt werden.

Durch entsprechendes Einstellen der Salzkonzentration und des pH-Wertes kann zudem ein dauerhaftes Binden kurzer einzelsträngiger Nukleinsäuren im Inneren der erfindungsgemäßen Vesikel verhindert werden.

Ein besonders bevorzugtes Verfahren zum Abtrennen einer Nukleinsäure umfasst die Schritte:
a) Inkontaktbringen einer die abzutrennende Nukleinsäure enthaltenden Lösung mit einem erfindungsgemäßen Vesikel wie oben beschrieben, das vorzugsweise Polylysin enthält und dessen Membran einen Porenbildner enthaltend die *β*-Fass-Domäne eines FhuA-Proteins enthält;
b) Eindringenlassen der abzutrennenden Nukleinsäure ins Innere des Vesikels; dies kann insbesondere bei Raumtemperatur geschehen;
c) Abtrennen der erfindungsgemäßen Vesikel von der die abzutrennende Nukleinsäure ursprüngliche enthaltenden Lösung; dies kann beispielsweise durch Zentrifugation und/oder Filtration geschehen;
d) Einstellen der Salzkonzentration, um Nukleinsäuren aus dem erfindungsgemäßen Vesikel abzutrennen, die eine geringere Größe als die abzutrennende Nukleinsäure besitzen (beispielsweise PCR-Primer), und Abtrennen der so freigesetzten Nukleinsäuren; und
e) Freisetzen der abgetrennten Nukleinsäure durch
   a. Zerstören des Vesikels durch Anlegen einer Scherkraft,
   b. Auflösen des Vesikels durch Ethanolzugabe und/oder
   c. durch Zugabe hoher NaCl-Konzentrationen und anschließendem Entfernen der Salze über eine herkömmliche Chromatographie-Säule.

Ein solches Verfahren kann innerhalb von 30 - 45 Minuten und damit im Vergleich zu herkömmlichen Verfahren ungewöhnlich schnell abgeschlossen werden.

Die Verwendung einer Poreneinheit nach einer der zuvor beschriebenen Arten zum Bilden einer Pore in einem Vesikel, dessen Membran ein amphiphiles Copolymer wie oben beschrieben enthält, ist ebenfalls erfindungsgemäß bevorzugt.

Bevorzugte Ausführungsformen der Erfindung werden nachfolgend anhand der Figur sowie der beigefügten Beispiele weiter erläutert. Es stellen dar:
- Fig. 1: Schematische Darstellung der Struktur eines erfindungsgemäßen Vesikels;
- Fig. 2: Fluoreszenzmessung von 500 nm bis 610 nm nach Zugabe von 60 µl SYBR Gold zu den Vesikeln mit und ohne DNA, s. Beispiel 3 Verfahren 1;
- Fig. 3: Fluoreszenzmessung von 500 nm bis 610 nm nach Zugabe von 60 µl SYBR Gold zu den Vesikeln mit und ohne DNA, s. Beispiel 3 Verfahren 1;
- Fig. 4: Fluoreszenzmessung von 500 nm bis 610 nm nach Zugabe von 60 µl SYBR Gold zu den Vesikeln mit und ohne DNA, s. Beispiel 3. Verfahren 2;
- Fig. 5: Fluoreszenzmessung von 500 nm bis 610 nm nach Zugabe von 60 µl SYBR Gold zu den Vesikeln mit und ohne DNA, s. Beispiel 3 Verfahren 3; und
- Fig. 6: Fluoreszenzmessung von 500 nm bis 610 nm nach Zugabe von 60 µl SYBR Gold zu den Vesikeln mit und ohne DNA, direktes Auflösen.

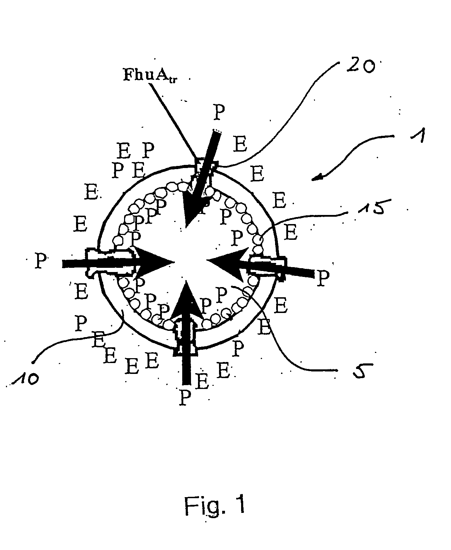

In Fig. 1 ist schematisch der Aufbau und die Wirkungsweise eines erfindungsgemäßen Vesikels zum Abtrennen einer geladenen Substanz dargestellt. Das Vesikel 1 besitzt eine Membran 10 aus PMOXA-PDMS-PMOXA Triblock-Copolymer. Das Vesikel 2 ist im wesentlichen kugelförmig und besitzt ein Vesikel-Inneres 5. Das Vesikel-Innere 5 ist frei von Feststoffen.

In der Membran 10 sind vier Poreneinheiten 20 enthalten, die jeweils einen Kanal zum Eintretenlassen einer Substanz (Pfeile) ins Vesikel-Innere 5 bilden. Die Poreneinheiten 20 sind FhuAₜᵣ-Proteine.

Auf der Innenseite 12 der Membran 10 ist ein Polyelektrolyt 15 angeordnet. Der Polyelektrolyt 15 hat eine zum Binden der geladenen Substanz geeignete Ladung. Wenn die zu bindende Substanz eine Nukleinsäure ist, dann ist der Polyelektrolyt vorzugsweise Polylysin.

Die geladene Substanz dringt durch eine Pore 20 in der Membran 10 des Vesikels 1 in das Vesikel-Innere 5 ein. Dort bindet sie an den Polyelektrolyten 15.

Zum Abgeben der geladenen Substanz aus dem Vesikel 1 wird das Vesikel 1 durch Anlegen einer Scherkraft (nicht dargestellt) zerstört. Das Vesikel 1 kann auch durch Ethanolzugabe aufgelöst und die gebundene Substanz auf diese Weise freigesetzt werden. Ein dritter Weg zum Freisetzen der gebundenen Substanz ist die Zugabe geeigneter Ionen, so dass die geladene Substanz vom Polyelektrolyten 15 gelöst wird. Die beiden letztgenannten Verfahren vermeiden es, die gebundene Substanz einer starken mechanischen Beanspruchung auszusetzen.

### Beispiel 1: Herstellung von FhuAₜᵣ:

Bakterienstamm und Wachstumsbedingunen: Es werden der E. *coli*-Stamm DH5*α* verwendet. Die Zellen werden bei 37 °C in LB-Medium (10 g/l Trypton, 5 g/l Hefeextrakt, 10 g/l NaCl) unter Schütteln mit 200 rpm gezüchtet. Es wird Ampicillin bis zu einer Endkonzentration von 100 µg/ml zugegeben und die optische Dichte bei 578 nm überwacht. Wenn die optische Dichte bei 578 nm einen Wert von 0,9-1 erreicht, wird Anhydrotetracyclin (200 µg/l Zellkultur) zugegeben, um den tet-Promotor auf dem in den Bakterien enthaltenen Plasmid zu induzieren. Die Zellen werden weitere 3 h (3 Stunden) inkubiert.

Plasmid: Das verwendete Plasmid ist pFHUA, ein pASK-IBA3-Derivat (IBA-Labortechnik, Göttingen). Das Wildtyp-Gen wurde erhalten vom Biozentrum Basel (Braun, M., Killmann, H., and Braun, V., The ß-barrel domain of FhuAΔ5-160 is sufficient for TonB-dependent FhuA activities of Escherichia coli, Mol. Microbiol. (1998)). Eine EcoRI-Restriktionsstelle wird im Wildtyp-Gen an der Aminosäureposition 129 (FhuA Δ1-129) und an der Aminosäureposition 160 (FhuA Δ1-160) eingebaut. Eine Xhol-Restriktionsstelle wurde in beiden Fällen an den Enden der Gene eingebaut mit Hilfe der PCR-Primer SEQ ID NO 1 und SEQ ID NO 2. Das amplifizierte DNA-Fragment wurde durch Agarose-Gelelektrophorese und mit QIAQuick-Kits (Qiagen) aufgereinigt, mit EcoRI (New England Biolabs) und Xhol (New England Biolabs) verdaut, erneut über Agarose-Gelektrophorese gereinigt und mit dem linearisierten pASK-IBA3-Plasmid (geschnitten mit EcoRI und Xhol) ligiert und durch Standard-Klonierverfahren in DH5*α* transformiert.

**Tabelle 1: PCR-Mischung für FhuAₜᵣ:**

| Substanz | Volumen (µl) |
|---|---|
| ddH₂O | 83.4 |
| 10x Puffer(Taq Puffer) | 10 |
| DNTP(10mM) | 2 |
| Primer SEQ ID NO 1(20 mM) | 1.3 |
| Primer SEQ ID NO 2(20mM) | 1.3 |
| Plasmid mit FhuA (Wildtyp-Gen) | 1 |
| Taq DNA Polymerase (5U/µl) | 1 |
| Summe | 100 |

**Tabelle 2: PCR-Bedingungen**

| Temperatur (°C) | Dauer (min) | Anzahl Zyklen |
|---|---|---|
| 94 | 3 | 1 |
| 94 | 1 | 31 |
| 68 | 1 | 31 |
| 72 | 3 | 31 |
| 72 | 10 | 1 |
| 8 | unbegrenzt | 1 |

**Tabelle 3: Verdau des Plasmids pASK-IBA3**

| Substanz | Volumen (µl) |
|---|---|
| ddH₂O | 68.16 |
| 10x Puffer (NEB Buffer 2) | 10 |
| Plasmid pASK-IBA3 | 20 |
| EcoR I (20U) | 0.42 |
| Xho I (20U) | 0.42 |
| BSA | 1 |
| Summe | 100 |

**Tabelle 4: Verdau des PCR-Produkts**

| Substanz | Volumen (µl) |
|---|---|
| ddH₂O | 16 |
| 10x Puffer (NEB Buffer 2) | 10 |
| PCR Product | 70 |
| EcoR I (20U) | 2 |
| Xho I (20U) | 2 |
| Summe | 100 |

**Tabelle 5: Verdau-Bedingungen**

| Temperatur | Dauer |
|---|---|
| 37°C (XhoI) | 4h |
| 37°C (EcoRI) | 4h |

**Tabelle 6: Ligation von FhuAₜᵣ in pASK-IBA3, um pFHUA zu erhalten**

| Substanz | Volumen (µl) |
|---|---|
| ddH₂O | 5 |
| 10x Puffer (Ligase-Puffer) | 2.5 |
| Plasmid pASK-IBA3 | 4 |
| Insert (FhuAₜᵣ) | 12 |
| Ligase (T4 DNA Ligase) | 1.5 |
| Summe | 25 |

Extraktion und Isolierung von FhuAₜᵣ: Nach Überexpression von FhuA wurden die DH5*α*-Zellen geerntet und 10 min lang bei 2800 Upm, 4 °C zentrifugiert. Der Überstand wurde verworfen. Zu 1 g des Zellpellets wird 5 ml Lysepuffer (50 mM Na-Phosphatpuffer, 100 mM NaCl, 2 mM EDTA und DNase I, pH 7,5) zugegeben. Die Mischung wird zweimal mit einer French Press behandelt. Das Lysat wird 10 min lang bei 7000 Upm abzentrifugiert. Der Überstand wird in einer Ultrazentrifuge bei 18 °C, 30000 Upm 40 min lang abzentrifugiert.

Das dabei entstehende Pellet wird in 20 ml Vorextraktionspuffer 1 (20 mM Na-Phosphatpuffer, 0.3% octyl-POE (n-Octyl-oligo-oxyethylen, Alexis) aufgelöst und homogenisiert, wobei zuerst ein Mixer verwendet wird und anschließend das Detergens zugegeben wird. Die Mischung wird 50 min lang bei 40 °C inkubiert und anschließend wie zuvor beschrieben in der Ultrazentrifuge abzentrifugiert.

Das dabei entstehende Pellet wird in 20 ml Vorextraktionspuffer 2 (20 mM Na-Phosphatpuffer (pH 7,5), 0.5% octyl-POE) aufgelöst und homogenisiert, wobei zuerst ein Mixer verwendet wird und anschließend das Detergens zugegeben wird. Die Mischung wird 50 min lang bei 40 °C inkubiert und anschließend wie zuvor beschrieben in der Ultrazentrifuge abzentrifugiert.

Das entstehende Pellet wird mit 20 ml Extraktionspuffer (20mM Na-Phosphatpuffer, 3% octyl-POE) versetzt und 40-60 min bei 37 °C inkubiert. Nach erneuter Ultrazentrifugation wie zuvor beschrieben erhält man FhuA im Überstand. Die Probe von FhuA wird auf einem SDS-Gel analysiert. Das FhuA wird durch Dialyse gegen 1 Vol.-% octyl-POE in Na-Phosphatpuffer pH 7,5 gewonnen und kann in dieser Form gelagert werden.

### Beispiel 2: Herstellen erfindungsgemäßer Vesikel

### Beispiel 2.1: Direktes Auflösen

50 mg des ABA (PMOXA-PDMS-PMOXA) Triblock-Copolymers (Nardin, C., Widmer, J., Winterhalter, M., and Meier, W., Amphiphilic block copolymer nanocontainers as bioreactors, Eur. Phys. J. (2001)) wurden in 4,95 ml PBS (0.37M NaCl, 27mM KCl, 43 mM Na₂HPO₄ 8H₂O, 14 mM KH₂PO₄ 2H₂O, pH 7,3) aufgelöst und über Nacht gerührt, um selbstorganisierte Vesikel zu bilden. Diese wurden dreimal extrudiert mit einem 0,4 µm -Filter (Millipore), um Vesikel einheitlicher Größe zu erhalten.

Um die Membranen der Vesikel zur Aufnahme von FhuA bereit zu machen, wird Triton X-100 (5% Endkonzentration) zugefügt. Etwa 80 µl FhuA werden zu der Mischung zugegeben und 3 h lang inkubiert. Detergenzien können die Vesikel destabilisieren, deshalb wurden zum Abtrennen des Detergens Biobeads (Rigaud, J.L., Paternostre, M.T., and Bluzat, A., Mechanisms of membrane protein insertion into liposomes during reconstitution procedures involving the use of detergents 2.Incorporation of the light-driven proton pump bacteriorhodopsin, Biochemistry (1988)) verwendet und 5-6 h lang unter langsamem Rühren inkubiert. Proben werden durch Sepharose 4B aufgereinigt, um nicht-rekonstituiertes FhuA und andere Verunreinigungen zu entfernen. Die Vesikel werden durch centricon YM-30 (Millipore) aufgereinigt.

### Beispiel 2.2: Ethanol-Verfahren

50 mg des ABA (PMOXA-PDMS-PMOXA) Triblock-Copolymers wurden in 250 µl Ethanol (99,8%) einige Minuten lang aufgelöst, um eine klare Lösung zu erhalten. Von oben wird die Lösung tropfenweise in 4,95 ml PBS (0,37 M NaCl, 27 mM KCl, 43 mM Na₂HPO₄ 8 H₂O, 14 mM KH₂PO₄ 2H2O, pH 7,3) zugegeben. Gleichzeitig werden 80 µl FhuA-Lösung langsam zugegeben. Die Mischung wird 3-4 h lang gerührt. Dreimal wurde extrudiert mit einem 0,4 µm-Filter (Millipore), um Vesikel einheitlicher Größe zu erhalten. Um das Detergens abzutrennen werden Bio-beads wie in Beispiel 2.1 verwendet, das übrige Verfahren verläuft wie in Beispiel 2.1.

### Beispiel 3: Herstellen von DNA-bindenden Vesikeln

50 mg des ABA (PMOXA-PDMS-PMOXA) Triblock-Copolymers werden in 4,95 ml Polylysin-Lösung (0,5 mg/ml der Polylysin-Lösung; Mol.-Gewicht 15000-30000 Sigma in 5 ml PBS)aufgelöst und wie in Beispiel 2.1 oder 2.2 beschrieben weiterbehandelt.

Nach dem Konzentrieren durch centricon YM-30 (Millipore) wurde zu der Suspension 50 µl DNA (60mer, 20 pmol) zugegeben und bei Raumtemperatur 30 min lang inkubiert.

Drei weitere Verfahren wurden entwickelt, um DNA abzutrennen, die nicht in den erfindungsgemäßen Vesikeln gebunden war:

### Verfahren 1:

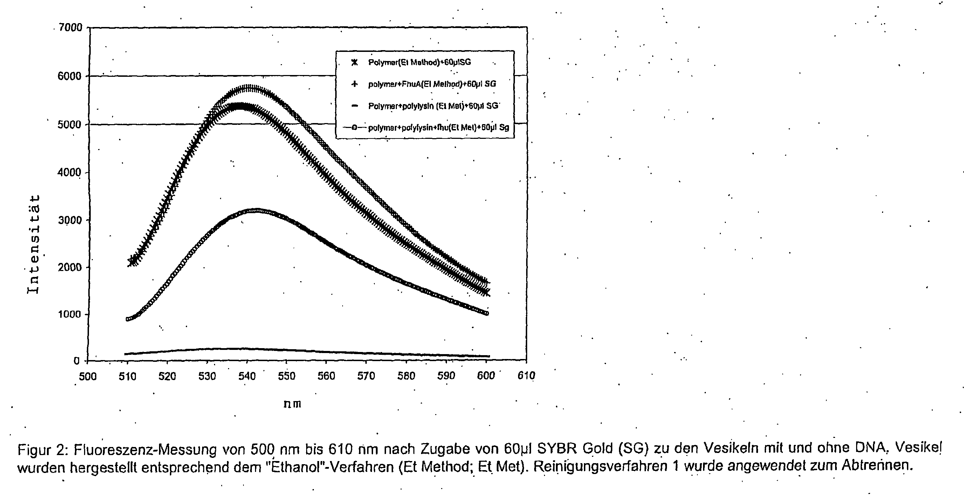

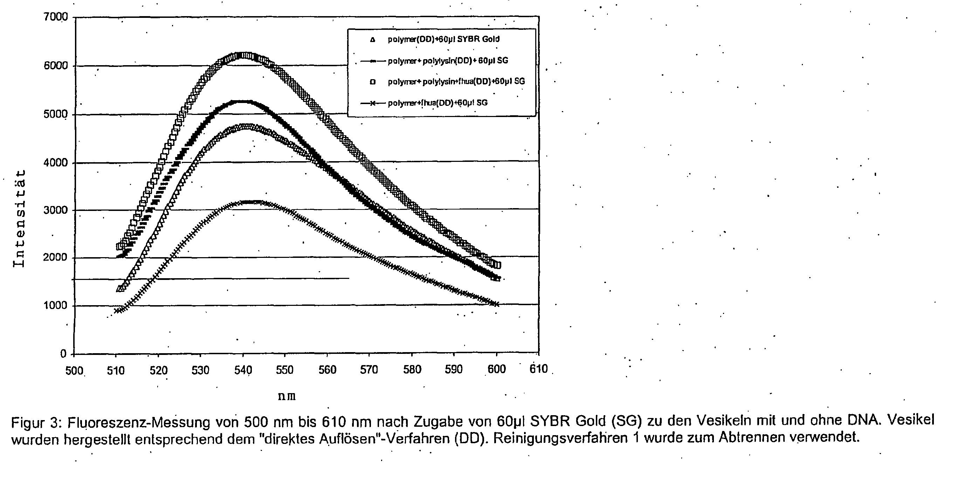

Die Suspension wurde sechsmal mit centricon YM-30 (Millipore) und PBS konzentriert und wieder verdünnt. Nach diesen wiederholten Konzentrierungs- und Waschschritten wurde 1 ml als Probe weiterverwendet und der Gehalt an in Vesikeln eingeschlossener DNA durch Zugeben von 60 µl SYBR Gold (1x Färben; Molecular Probes, Leiden, Niederlande) bestimmt (s. Fig. 2, 3).

### Verfahren 2:

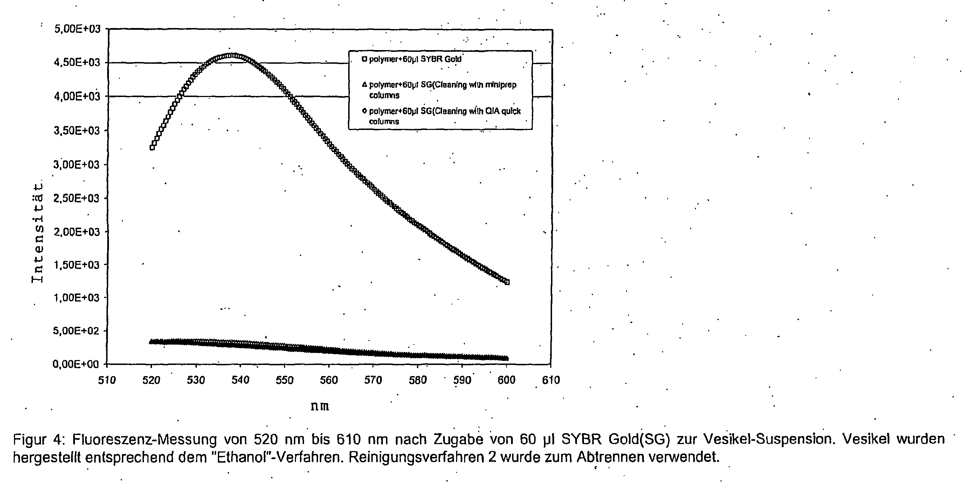

In diesem Verfahren wurden Qiagen-Reinigungskits (Qia Miniprep, Qiaquick) verwendet, um die DNA innerhalb der Vesikel von der außerhalb der Vesikel abzutrennen. Die eluierte Lösung mit den Vesikeln wurde zentrifugiert (3000 g, 2 min) und der Gehalt an in Vesikeln eingeschlossener DNA durch Zugeben von 60 µl SYBR Gold (1 x Färben; Molecular Probes, Leiden, Niederlande) bestimmt (s. Fig. 4).

### Verfahren 3:

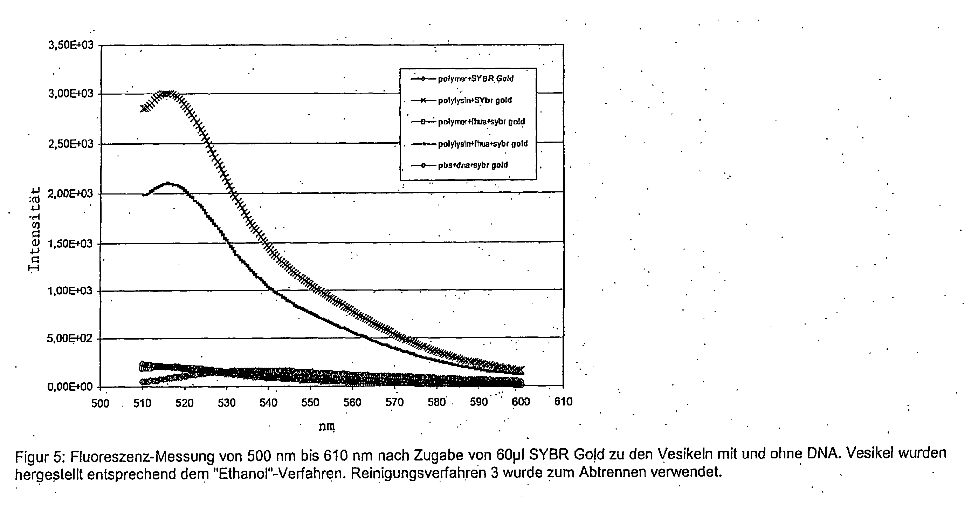

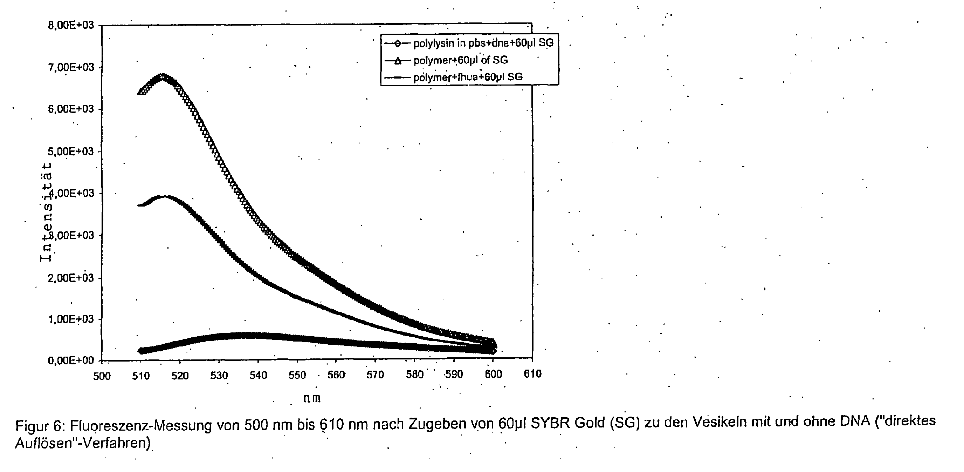

Zum Entfernen ungebundener DNA, die nicht in die Vesikel eingedrungen war, wurde zusätzlich eine Sepharose 4B-Säule verwendet. Der Gehalt an in Vesikeln eingeschlossener DNA wurde durch Zugeben von 60 µl SYBR Gold (1x Färben; Molecular Probes, Leiden, Niederlande) bestimmt (s. Fig. 5, 6).

## Patentansprüche

1. Vesikel zum Binden einer Substanz
- mit einer Membran enthaltend amphiphile Moleküle,
- einer in der Membran enthaltenen porenbildenden Poreneinheit, um den Zugang zum Vesikel-Inneren zu ermöglichen,
wobei das Vesikel im Vesikel-Inneren eine Bindungssubstanz enthält zum Binden der zu bindenden Substanz, und wobei die Bindungssubstanz ein Polyelektrolyt oder eine Oligosäure oder Oligobase aus 10-100 Säure- bzw. Basengrundeinheiten ist und nicht durch die von der Poreneinheit gebildete Pore diffundieren kann, und wobei die Poreneinheit ein Protein oder einen Proteinteil enthält.

2. Vesikel nach Anspruch 1, **dadurch gekennzeichnet, dass** die Bindungssubstanz eingerichtet ist zum Vermitteln einer ionischen Bindung, einer Wasserstoffbrückenbindung und/oder einer hydrophoben Wechselwirkung.

3. Vesikel nach einem der vorherigen Ansprüche, **dadurch gekennzeichnet, dass** die Poreneinheit ein Protein oder einen Proteinteil enthält, das/der ausgewählt ist aus der Gruppe bestehend aus
a) einem Transmembranprotein,
b) einem Transmembranprotein mit einer alpha-helikalen Transmembran-Struktur,
c) einem Transmembranprotein mit einer beta -Fass-Transmembran-Struktur,
d) einer Transmembran-Struktur eines Transmembranproteins, und
e) einem Protein, dass eine zu einer Transmembranstruktur eines der Proteine gemäss a), b), c) und/oder d) strukturhomologe Struktur besitzt.

4. Vesikel nach einem der vorherigen Ansprüche, **dadurch gekennzeichnet, dass** die Poreneinheit einen inneren Porendurchmesser von mehr als 0,1 nm Breite besitzt.

5. Vesikel nach einem der vorherigen Ansprüche, **dadurch gekennzeichnet, dass** die Poreneinheit eine enantioselektive Pore bildet.

6. Vesikel nach einem der vorherigen Ansprüche, **dadurch gekennzeichnet, dass** das Vesikel im Vesikel-Inneren ein positiv geladenes Oligomer oder Polymer besitzt.

7. Vesikel nach Anspruch 6, **dadurch gekennzeichnet, dass** das Vesikel im Vesikel-Inneren Polylysin enthält.

8. Verwendung eines Vesikels nach einem der vorherigen Ansprüche zum Binden einer Substanz.

9. Verwendung nach Anspruch 8, wobei die zu bindende Substanz eine Nukleinsäure ist.

10. Verfahren zum Binden einer Nukleinsäure, umfassend das Inkontaktbringen der zu bindenden Nukleinsäure mit einem Vesikel nach einem der Ansprüche 1 bis 7.

11. Verfahren zum Abgeben einer Nukleinsäure, umfassend die Schritte:
a) Binden einer Nukleinsäure in einem Vesikel durch ein Verfahren nach Anspruch 10, und
b) anschliessend Freisetzen der gebundenen Nukleinsäure durch Anlegen einer Scherspannung an das Vesikel und/oder Auflösen des Vesikels, und/oder durch Zugabe eines Salzes.

## Claims

1. A vesicle for binding a substance
- with a membrane containing amphiphilic molecules,
- a pore-forming pore complex for allowing access to the inside of the vesicle, wherein the vesicle contains a binding substance on the inside of the vesicle for binding the substance to be bonded, and wherein the binding substance is a polyelectrolyte or an oligo-acid or oligo-base of 10-100 acid units, respectively base units and cannot diffuse through the pores formed by the pore complex, and wherein the pore complex contains a protein or a protein part.

2. The vesicle according to claim 1, **characterized in that** the binding substance is adapted to promote an ionic bonding, a hydrogen bond and/or a hydrophobic interaction.

3. The vesicle according to one of the afore-mentioned claims, **characterized in that** the pore complex contains a protein or a protein part chosen from a group formed by
a) a transmembrane protein
b) a transmembrane protein with an alpha-helical transmembrane structure,
c) a transmembrane protein with a beta barrel transmembrane structure,
d) a transmembrane structure of a transmembrane protein, and
e) a protein having a structure with a homologous structure to the transmembrane structure of one of the proteins according to a), b), c) and/or d).

4. The vesicle according to one of the afore-mentioned claims, **characterized in that** the pore complex has an inner pores diameter with a width of more than 0.1 nm.

5. The vesicle according to one of the afore-mentioned claims, **characterized in that** the pore complex forms an enantioselective pore.

6. The vesicle according to one of the afore-mentiond claims, **characterized in that** the vesicle has a positively loaded oligomer or polymer on the inside of the vesicle.

7. The vesicle according to claim 6, **characterized in that** the vesicle contains polylysine on the inside of the vesicle.

8. The use of a vesicle according to one of the afore-mentioned claims for binding a substance.

9. The use according to claim 8, wherein the substance to be bonded is a nucleic acid.

10. A method for binding a nucleic acid, comprising bringing the nucleic acid to be bonded in contact with a vesicle according to one of the claims 1 to 7.

11. A method for releasing a nucleic acid, comprising the steps of:
a) binding a nucleic acid in a vesicle by means of a method according to claim 10 and
b) subsequently releasing the bonded nucleic acid by applying a shear stress to the vesicle and/or dissolving the vesicle and/or by adding a salt.

## Revendications

1. Vésicule pour lier une substance
- avec une membrane contenant une molécule amphiphile,
- un complexe de pores, contenu dans la membrane, formant des pores pour permettre l'accès à l'intérieur de la vésicule,
où la vésicule contient une substance liante à l'intérieur de la vésicule pour lier la substance à lier et où la substance liante est un polyélectrolyte ou un oligo-acide ou une oligo-base de 10-100 unités et ne peut pas diffuser à travers les pores formées par le complexe de pores, et où le complexe de pores contient une protéine ou une partie de protéine.

2. Vésicule selon la revendication 1, **caractérisée en ce que** la substance liante est apte à promouvoir une liaison ionique, une liaison hydrogène et/ou une interaction hydrophobe.

3. Vésicule selon l'une quelconque des revendications précédentes, **caractérisée en ce que** le complexe de pores contient une protéine ou une partie de protéine choisie au sein du groupe formé par
a) une protéine transmembranaire
b) une protéine transmembranaire avec une structure transmembranaire alpha-hélicoïdale,
c) une protéine transmembranaire avec une structure transmembranaire en forme de tonneau-béta,
d) une structure transmembranaire d'une protéine transmembranaire et
e) une protéine ayant une structure homologue d'une structure transmembranaire de l'une des protéines selon a), b), c) et/ou d).

4. Vésicule selon l'une quelconque des revendications précédentes, **caractérisée en ce que** le complexe de pores possède un diamètre de pore interne de plus de 0,1 nm de large.

5. Vésicule selon l'une quelconque des revendications précédentes, **caractérisée en ce que** le complexe de pores forme des pores énantiosélectifs.

6. Vésicule selon l'une quelconque des revendications précédentes, **caractérisée en ce que** le vésicule possède un oligomère ou un polymère chargés positivement à l'intérieur de la vésicule.

7. Vésicule selon la revendication 6, **caractérisée en ce que** la vésicule contient une polylysine à l'intérieur de la vésicule.

8. Utilisation d'une vésicule selon l'une quelconque des revendications précédentes pour lier une substance.

9. Utilisation selon la revendication 8, où la substance à lier est un acide nucléique.

10. Méthode pour lier un acide nucléique comprenant la mise en contact des acides nucléiques à lier avec une vésicule selon l'une des revendications 1 à 7.

11. Méthode pour restituer un acide nucléique comprenant les étapes suivantes :
a) Liaison d'un acide nucléique dans une vésicule au moyen d'une méthode selon la revendication 10, et
b) dans un second temps, libération de l'acide nucléique lié en appliquant une contrainte de cisaillement au sein de la vésicule et/ou par dissolution de la vésicule et/ou par ajout d'un sel.


### SEQUENCE LISTING

<110> International University Bremen GmbH
<120> Vesikel zum Abtrennen von Substanzen aus flüssigen Medien
<130> IA 820-01EP
<160> 2
<170> PatentIn version 3.1
<210> 1
   <211> 39
   <212> DNA
   <213> Artificial Sequence
<220>
   <223> forward primer for PCR amplification
<220>
   <221> misc_feature
   <222> (4)..(9)
   <223> BsaI restriction site
<220>
   <221> misc_feature
   <222> (10)..(15)
   <223> EcoRI restriction site
<220>
   <221> misc_feature
   <222> (19)..(21)
   <223> "start" codon
<400> 1
   atgggtctcg aattccggat gcgcgctgaa attatgcgt 39
<210> 2
   <211> 40
   <212> DNA
   <213> Artificial Sequence
<220>
   <223> reverse primer for PCR amplification
<220>
   <221> misc_feature
   <222> (4)..(10)
   <223> Nco restriction site
<220>
   <221> misc_feature
   <222> (11)..(16)
   <223> XhoI restriction site
<220>
   <221> misc_feature
   <222> (17)..(19)
   <223> "stop" codon
<400> 2
   catgccatgg ctcgagttag aaacggaagg ttgcggttgc 40